(19) Europäisches Patentamt
European Patent Office
Office européen des brevets

(11) Numéro de publication : **0 080 913**
**B1**

# (12) FASCICULE DE BREVET EUROPÉEN

(45) Date de publication du fascicule du brevet : **13.03.85**

(21) Numéro de dépôt : **82401994.7**

(22) Date de dépôt : **28.10.82**

(51) Int. Cl.4 : **C 07 C 68/02**, C 07 C 69/96, B 01 J 31/02

## (54) Procédé de synthèse de chloroformiate de chlorométhyle.

(30) Priorité : **10.11.81 US 320006**
**22.03.82 US 360471**

(43) Date de publication de la demande : **08.06.83 Bulletin 83/23**

(45) Mention de la délivrance du brevet : **13.03.85 Bulletin 85/11**

(84) Etats contractants désignés : **BE CH DE FR GB IT LI NL SE**

(56) Documents cités :
**EP-A- 0 032 245**
**EP-A- 0 040 153**
**DE-B- 2 131 555**
**FR-A- 2 296 615**
**FR-A- 2 381 739**

(73) Titulaire : **SOCIETE NATIONALE DES POUDRES ET EXPLOSIFS**
**12, quai Henri IV**
**F-75181 Paris Cedex 04 (FR)**

(72) Inventeur : **Olofson, Roy Arne, Dr.**
**529 Cricklewood Drive**
**State College Pennsylvania 16801 (US)**
Inventeur : **Martz, Jonathan Thomas, Dr.**
**1441 Whightman Street**
**Pittsburgh, PA.15217 (US)**

**EP 0 080 913 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

## Description

L'invention concerne la fabrication du chloroformiate de chlorométhyle qui est une matière intéressante pour de nombreuses synthèses organiques mais qui n'est pas accessible de façon aisée à l'échelle industrielle.

La synthèse de chloroformiates α-chlorés de formule générale :

$$R - CHCl - O - \underset{\underset{O}{\|}}{C} - Cl$$

où R est un substituant aliphatique ou aromatique est une entreprise très difficile si l'on s'impose de ne pas additionner un atome de chlore supplémentaire sur le radical R, au cours de ladite synthèse.

Müller dans les Liebig's Annalen der Chemie de 1890, volume 257, page 50 et suivantes, a proposé un procédé qui est encore le seul connu et utilisé de nos jours. Ce procédé consiste à chlorer photolytiquement le chloroformiate correspondant, non substitué en α. On obtient malheureusement, à côté du produit recherché, de nombreux sous-produits plus chlorés que nécessaire. Müller en a ainsi dénombré pas moins de cinq dans le cas du chloroformiate d'éthyle qu'il a étudié.

Or, la présence de ces sous-produits est extrêmement gênante en raison de l'application principale qui est faite desdits chloroformiates à savoir leur transformation en carbonates notamment utiles en synthèse pharmaceutique fine pour obtenir, par exemple, des acylals d'acide pénicilline.

Une distillation du produit de la réaction est donc indispensable bien que délicate en raison de la présence de nombreux sous-produits.

Il existe une autre publication ancienne, le brevet allemand 121 223 de 1901, décrivant la synthèse de chloroformiate de tétrachloro-1,2,2,2-éthyle et du chloroformiate d'α-chloro benzyle, par phosgénation respectivement du chloral et du benzaldéhyde, en présence d'une quantité stœchiométrique d'une amine tertiaire n'appartenant pas à la série pyridinique.

S'il vient à l'idée de tenter dans les mêmes conditions la phosgénation d'autres aldéhydes moins particuliers que les précédents, par exemple l'acétaldéhyde, on observe la formation de nombreux complexes et sous-produits à côté du chloroformiate d'α-chloréthyle qui n'est obtenu qu'avec un rendement médiocre, ce qui rend le procédé inintéressant à l'échelle industrielle.

Par ailleurs, s'il vient encore à l'idée de tenter la phosgénation avec une amine tertiaire aliphatique, par exemple la triéthylamine, on constate essentiellement la destruction de cette amine avec seulement formation d'une très faible quantité du chloroformiate dérivé.

Il existe donc un besoin non satisfait en un procédé de fabrication de chloroformiates α-chlorés purs, si possible avec un bon rendement, qui permette enfin d'assurer à ces produits de structure très simple le développement qu'ils méritent, notamment en tant qu'intermédiaires.

Il a récemment été proposé un procédé de fabrication de chloroformiates α-chlorés exempts de sous-produits de substitution ultérieure à partir de matières premières peu onéreuses et conduisant à des rendements excellents. Ce procédé, décrit dans la demande de brevet irlandais 969/81, consiste à phosgéner un aldéhyde RCHO en présence de catalyseurs de manière à obtenir le chloroformiate α-chloré RCH Cl OCO Cl. Toutefois ce procédé ne peut être appliqué tel qu'il est décrit au formol HCHO et ne permet pas l'obtention du chloroformiate de chlorométhyle $CH_2Cl$ OCO Cl.

S'agissant du chloroformiate de chlorométhyle on sait par ailleurs qu'on peut l'obtenir par chloruration du chloroformiate de méthyle ou du formiate de méthyle. Matzner et coll., dans Chemical Review 64, page 646, (1964) citent plusieurs références sur ces procédés connus, mais il s'agit toujours de synthèses délicates conduisant à de nombreux sous produits difficiles à séparer du produit recherché.

La présente invention concerne un procédé de synthèse avec un rendement élevé du chloroformiate de chlorométhyle par phosgénation du formol caractérisé en ce que l'on introduit du formol gazeux monomère préalablement desséché dans un réacteur contenant du phosgène et un catalyseur choisi dans le groupe constitué par les amides substitués, les urées ou thiourées tétrasubstituées, les phosphoramides totalement substitués à l'azote, les halogénures d'ammoniums quaternaires dont les substituants comportent au total au moins 16 atomes de carbone et préférentiellement ceux dont chaque substituant comporte au moins 4 atomes de carbone, les halogénures alcalins ou alcalinoterreux associés à un complexant de leurs cations, ainsi que les produits de réaction de ces catalyseurs avec le phosgène, et en ce que l'on effectue la réaction en absence totale d'eau et d'acide chlorhydrique à une température comprise entre − 10 °C et + 60 °C.

Selon une réalisation particulière de l'invention la réaction du formol sur le phosgène en présence d'un catalyseur est effectuée dans un solvant choisi dans le groupe constitué par le toluène, le chlorure de méthylène, le chloroforme, le tétrachlorure de carbone.

Selon une réalisation préférée de l'invention le catalyseur est choisi dans le groupe constitué par le chlorure de benzyl tributyl ammonium, le chlorure de potassium associé à un éther couronne ou à un cyptate capables de complexer son cation, la tétrabutyl urée phosgénée.

Le procédé selon l'invention consiste à effectuer dans un réacteur, à une température comprise entre

– 10 °C et + 60 °C la réaction du formol gazeux sec à l'état de monomère sur le phosgène en présence d'un catalyseur et en absence totale d'eau et d'acide chlorhydrique.

Selon l'invention le formol doit être parfaitement sec et en totalité à l'état de monomère. Avant d'effectuer la réaction il convient donc de sécher le formol et d'autre part, en règle générale, de le dépolymériser, le formol ne pouvant être conservé sous forme monomère mais seulement sous forme soit de son trimère le trioxanne soit d'un polymère linéaire de formule générale $(CH_2O)_n$, le paraformaldéhyde, n étant un entier compris le plus souvent entre 6 et 100. Le formol est desséché au dessiccateur en présence d'un bon agent desséchant comme le pentoxyde de phosphore. L'opération de dessiccation du formol peut être effectuée indifféremment avant ou pendant la dépolymérisation mais en tout état de cause avant introduction dans le réacteur de phosgénation. L'opération de dessiccation a une importance fondamentale dans le procédé selon l'invention et doit être parfaitement exécutée. En effet toute trace d'humidité entraîne la repolymérisation du formol monomère et fait chuter le rendement de la phosgénation, car seul le formol monomère est susceptible de réagir avec le phosgène. Le formol monomère est obtenu de manière connue en soi par exemple par dépolymérisation thermique dans le cas du paraformaldéhyde ou par dépolymérisation en présence de catalyseurs dans le cas du trioxanne. La dépolymérisation peut avoir lieu pendant dessiccation du formol polymère ou après dessiccation du formol polymère. Le formol sec à l'état de monomère est alors introduit dans un réacteur parfaitement sec contenant le catalyseur et le phosgène.

Le terme « catalyseur » doit, dans la présente description, être pris dans une acception restrictive. Le composé ajouté en tant que catalyseur est indispensable au déroulement correct de la réaction, ne participe pas directement à la réaction et est utilisé en quantités relativement faibles par rapport au formol : en ce sens, il est bien un catalyseur ; mais, contrairement à ce qui est communément admis pour les catalyseurs, il n'est pas toujours réutilisable pour une autre réaction une fois que l'on a arrêté l'arrivée de phosgène, et la demanderesse n'a pas d'explications théoriques à proposer pour ce phénomène.

On a pu trouver une définition commune à un certain nombre de catalyseurs convenant bien dans le cadre de l'invention. Ces catalyseurs sont des substances organiques ou minérales capables, dans un milieu contenant du formol, du phosgène et, éventuellement, un solvant, de générer une paire d'ions dont l'un est un anion halogénure et l'autre un cation suffisamment séparé dudit anion halogénure pour impartir à ce dernier un pouvoir nucléophile lui permettant d'attaquer la fonction aldéhyde du formol. Comme catalyseurs selon l'invention entrant dans cette définition on peut notamment citer les substances suivantes en tant que telles ou sous forme de leur produit de réaction avec le phosgène : les amides substitués, les urées et thiourées tétra substituées, les phosphoramides totalement substitués à l'azote, les halogénures d'ammoniums quaternaires dont les substituants comportent au total au moins 16 atomes de carbone et préférentiellement ceux dont chaque substituant comporte au moins 4 atomes de carbone et les halogénures alcalins ou alcalino-terreux associés à un complexant de leur cation. De préférence l'halogénure est le chlorure.

Comme il a été exposé plus haut un certain nombre de catalyseurs sont du type générateur d'anion halogénure soit directement, soit après réaction sur le phosgène.

Dans ce cas, le mécanisme général d'action du catalyseur est selon toute vraisemblance le suivant :

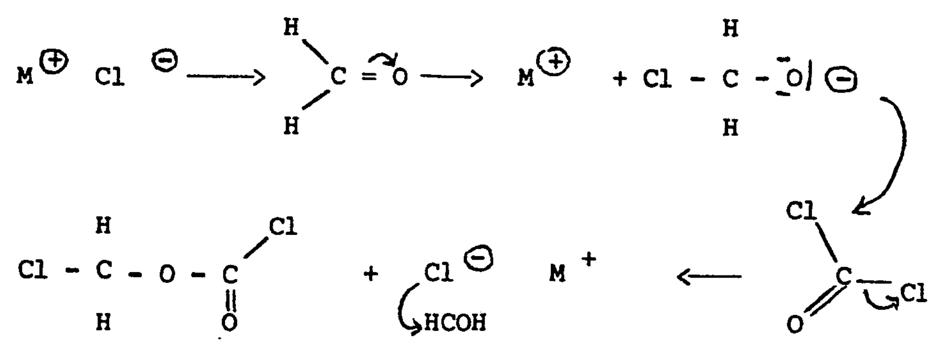

où $M^+$ représente un cation organique ou minéral, complexé ou non, présent en la forme dans le catalyseur dès le départ ou bien formé dès les premiers instants de la réaction par action du phosgène sur le catalyseur.

Ainsi $M^+$ peut être un cation métallique complexé ou un cation entièrement organique du type onium et on a par exemple :

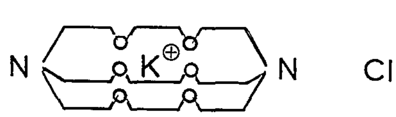

ou bien $M^+$ provient de la réaction plus ou moins avancée du phosgène sur la substance responsable de l'activité catalytique, comme par exemple dans la séquence :

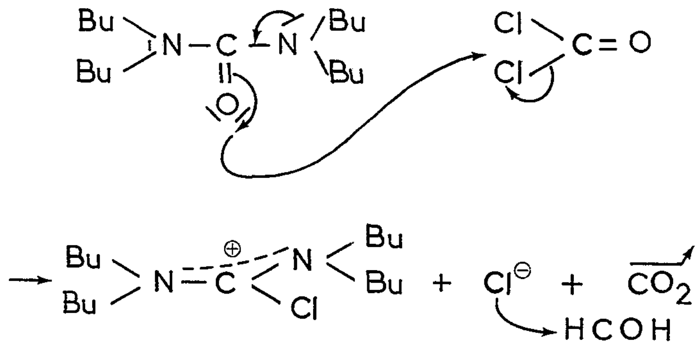

où donc $M^+$ est un volumineux cation chlorimonium.

On a observé que les résultats les plus intéressants sont obtenus avec les catalyseurs suivants : les amides substitués et plus particulièrement le diméthylformamide, les urées et thiourées tétra substituées et plus particulièrement les tétraalkyl (thio)urées telles que la tétrabutylurée et la tétraméthylurée, les phosphoramides totalement substituées à l'azote et plus particulièrement l'hexaméthylphosphotriamide, les halogénures d'ammoniums quaternaires dont tous les radicaux hydrocarbyl substituants comportent tous réunis au moins 16 atomes de carbone et de préférence au moins 4 atomes de carbone chacun, tels que le chlorure de tributyl benzyl ammonium, les halogénures alcalins ou alcalino-terreux associés à un complexant de leurs cations, tels que les chlorures alcalins ou alcalino-terreux et notamment le chlorure de potassium associés à un éther couronne tel que le 18 — couronne — 6 ou un cryptate tel que le (222) ou diaza-1,10 hexaoxa-4,7,13,16,21,24-bicyclo (8,8,8) hexacosane.

Naturellement dans ce dernier cas on associe de préférence un complexant formant avec le cation du chlorure métallique un complexe ayant une constante de stabilité élevée ce qu'il est très facile de faire compte tenu des nombreuses études effectuées sur le sujet telles que celle de Kappenstein parue dans le Bulletin de la Société Chimique de France, 1974, N° 1-2, pages 89-109 et celle de J. M. Lehn parue dans Structure and Bonding, volume 16, pages 2-64, Springer Verlag, (1974). Dans ce qui précède par halogénure on entend essentiellement un chlorure, un bromure ou un iodure, étant bien entendu qu'on préfère un chlorure, de manière à ce que même la première molécule de formol transformée grâce à l'action de l'halogénure provenant du catalyseur, soit transformée en chloroformiate de chlorométhyle.

Le taux de catalyseur employé est une caractéristique importante mais non fondamentale du procédé selon l'invention. En effet s'agissant d'un catalyseur particulièrement efficace, un taux de 0,5 à 10 % en moles, de préférence de 2 à 7 %, de catalyseur par rapport à la quantité molaire de phosgène utilisé doit être adopté. Par contre certains catalyseurs selon l'invention sont moins efficace et un taux de 1 à 50 % environ, de préférence de 5 à 40 %, soit un taux en moyenne plus élevé, doit être utilisé.

L'ordre d'introduction des réactifs dans le procédé selon l'invention est fondamental. Il est en effet obligatoire d'introduire le formol gazeux monomère dans le réacteur contenant le catalyseur et le phosgène de manière à ce que le formol réagisse immédiatement avec le phosgène sans avoir le temps de se repolymériser, la vitesse de réaction du formol sur le phosgène étant supérieure à la vitesse de polymérisation du formol dans les conditions opératoires. Le réacteur doit donc contenir au moins le catalyseur, le phosgène étant soit introduit en totalité dans le réacteur avant le début de la réaction, soit encore introduit en même temps que le formol dans un pied de cuve de phosgène et de catalyseur. Par contre il n'est pas possible, dans le cadre de la présente invention, de constituer un pied de cuve avec le formol et le catalyseur, et d'introduire le phosgène dans ce pied de cuve, en effet, en pareil cas, le formol polymériserait et la réaction de phosgénation ne serait quasiment plus possible.

La réaction de phosgénation est effectuée en milieu agité. Pendant l'introduction du formol la température du milieu réactionnel est de préférence maintenue entre − 10 °C et + 30 °C, de manière particulièrement préférée la température du milieu réactionnel est maintenue autour de 0 °C quand on commence à ajouter le formol et on la laisse remonter jusqu'à environ 20 °C à la fin de l'introduction du formol. Il peut être avantageux de terminer la réaction en chauffant le milieu réactionnel jusqu'à une température comprise entre 40 et 60 °C.

Le milieu réactionnel doit être parfaitement exempt de toute trace d'eau ou d'acide chlorhydrique de manière à éviter tout risque de repolymérisation du formol. A cette fin, avant réaction, le réacteur doit être purgé avec de l'air sec ou avec un gaz inerte sec.

Bien qu'il ne s'agisse pas là d'une mise en œuvre préférée de l'invention, la réaction peut être effectuée en présence d'un solvant. Il faut cependant veiller à éviter les solvants qui réagissent avec le phosgène en donnant de l'acide chlorydrique comme par exemple les alcools et les amines, ceux qui se dégradent en donnant de l'acide chlorydrique comme les cétones ou le tétrahydrofuranne, et enfin ceux qui sont difficiles à sécher comme les éthers. Si on le désire on pourra utiliser comme solvants le toluène ou les solvants aliphatiques chlorés comme le chlorure de méthylène, le chloroforme, le tétrachlorure de carbone. Toutefois, l'utilisation d'un solvant peut présenter dans certains cas un intérêt, car lorsque la réaction est conduite en présence d'un solvant, la température peut être maintenue entre 30 et 60 °C même pendant l'introduction du formol.

Les exemples qui suivent illustrent la mise en œuvre du procédé selon l'invention.

Exemple 1

L'appareillage est constitué par un réacteur en verre de 100 ml équipé d'un réfrigérant à carboglace, d'un thermomètre, d'un agitateur et d'un tube d'introduction de gaz.

Le réacteur est purgé à l'azote sec. On introduit alors 38 g (0,38 mole) de phosgène dans le réacteur contenant 3,3 g (0,010 6 mole) de chlorure de benzyl tributylammonium parfaitement sec.

La température du milieu étant maintenue aux environs de 0 °C on introduit, par le tube d'introduction qui plonge dans le phosgène, le formaldéhyde provenant d'un ballon contenant 18 g (0,6 mole) de paraformaldéhyde et 10 g de pentoxyde de phosphore $P_2O_5$, le ballon étant purgé à l'azote sec et chauffé à 150 °C. On poursuit l'introduction du formol pendant une demi-heure jusqu'à disparition totale du paraformaldéhyde, puis on laisse revenir le milieu réactionnel à 20 °C et on maintient sous agitation une heure à cette température. On élimine le phosgène résiduel par dégazage et on purifie le chloroformiate de chlorométhyle obtenu d'abord par évaporation sous vide puis par distillation sous pression atmosphérique des volatiles recueillis (température d'ébullition : 106 °C).

On obtient ainsi 20,7 g de produit parfaitement sec soit un rendement de 42 % par rapport au phosgène introduit. Le chloroformiate de chlorométhyle se caractérise en spectographie RMN par un singulet à 5,5 ppm.

Exemple 2

On opère comme décrit dans l'exemple 1 avec 10 g de phosgène, 10 g de paraformaldéhyde et 5 g de $P_2O_5$ en utilisant comme catalyseur de la tétra n-butyl urée phosgénée. Le catalyseur a été obtenu en phosgénant à 50 °C 1,5 g de tétra n-butyl urée selon le schéma réactionnel suivant :

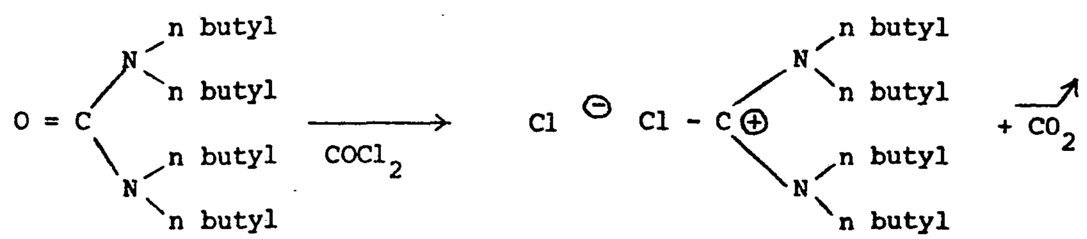


Après la fin de l'introduction du formaldéhyde, on porte le milieu réactionnel pendant une heure à 50 °C.

On obtient ainsi du chloroformiate de chlorométhyle avec un rendement calculé de 91,5 % par rapport au phosgène introduit. Ce rendement est déterminé par dosage RMN en utilisant du toluène comme étalon interne.

Exemple 3

On opère comme décrit dans l'exemple 2 avec 20 g de phosgène, 15 g de paraformaldéhyde et 10 g de $P_2O_5$ en utilisant comme catalyseur 1,3 g de chlorure de potassium associé à 0,4 g de cryptate (2.2.2.). On obtient ainsi du chloroformiate de chlorométhyle avec un rendement calculé de 63 % par rapport au phosgène introduit.

Exemple 4

L'appareillage est analogue à celui de l'exemple 1. Le réacteur est purgé à l'azote sec. On introduit 20 g de phosgène dans le réacteur contenant 1,3 g de chlorure de potassium associé à 0,4 g de cryptate (2.2.2.).

La température du milieu étant maintenue aux environs de 0 °C on introduit, par le tube de bullage qui plonge dans le phosgène, le formaldéhyde provenant d'un ballon contenant 15 g de paraformaldéhyde chauffé à 150 °C. Le ballon a été préalablement purgé à l'azote et le paraformaldéhyde a été séché sous vide de 0,1 mm Hg sur $P_2O_5$ dans un dessiccateur avant introduction dans le ballon de dépolymérisation. On poursuit l'introduction du formol pendant une demi-heure puis on chauffe le milieu réactionnel à 50 °C pendant une heure pour parfaire la réaction.

On obtient ainsi du chloroformiate de chlorométhyle avec un rendement calculé de 73 % par rapport au phosgène introduit.

Exemple 5

L'appareillage est analogue à celui de l'exemple 1. Le réacteur est purgé à l'azote sec. On introduit 40

ml de tétrachlorure de carbone anhydre comme solvant, 12 g de phosgène et comme catalyseur de la tétra n-butyl urée phosgénée obtenue comme décrit dans l'exemple 2 à partir de 1,5 g de tétra n-butyl urée.

On porte le milieu réactionnel à 40 °C et on introduit du formaldéhyde obtenu comme décrit à l'exemple 4 à partir de 3,8 g de paraformaldéhyde. Après introduction du formaldéhyde on maintient le milieu réactionnel pendant 2 heures à 40 °C.

On obtient ainsi du chloroformiate de chlorométhyle avec un rendement calculé de 65 % par rapport au formol introduit.

## Revendications

1. Procédé de synthèse du chloroformiate de chlorométhyle par phosgénation du formol caractérisé en ce que l'on introduit du formol gazeux préalablement desséché et à l'état de monomère dans un réacteur contenant du phosgène et un catalyseur choisi dans un groupe constitué par les amides substitués, les urées ou thiourées tétrasubstituées, les phosphoramides totalement substitués à l'azote, les halogénures d'ammoniums quaternaires dont les substituants comportent au total au moins 16 atomes de carbone, les halogénures alcalins ou les alcalino-terreux associés à un complexant de leurs cations, ainsi que les produits de réaction de ces catalyseurs avec le phosgène et en ce que l'on effectue la réaction en absence totale d'eau et d'acide chlorhydrique à une température comprise entre − 10 °C et + 60 °C.

2. Procédé selon la revendication 1 caractérisé en ce que ledit catalyseur est choisi dans le groupe constitué par les amides substitués, les urées ou les thiourées tétrasubstituées, les phosphoramides totalement substitués à l'azote, les halogénures d'ammoniums quaternaires dont chaque substituant comporte au moins quatre atomes de carbone, les chlorures alcalins ou alcalino-terreux associés à un complexant de leurs cations, ainsi que les produits de réaction de ces catalyseurs avec le phosgène.

3. Procédé selon la revendication 2 caractérisé en ce que ledit catalyseur est choisi dans le groupe constitué par le diméthylformamide, la tétrabutylurée, la tétraméthylurée, l'hexaméthylphosphotriamide, le chlorure de tributyl benzyl ammonium, le chlorure de potassium associé à l'éther 18-couronne-6, le chlorure de potassium associé au cryptate (2.2.2.).

4. Procédé selon l'une quelconque des revendications de 1 à 3 caractérisé en ce que l'agent desséchant utilisé pour dessécher le formol est le pentoxyde de phosphore.

5. Procédé selon l'une quelconque des revendications 1 à 4 caractérisé en ce que pendant l'introduction du formol la température du milieu réactionnel est maintenue entre − 10 °C et + 30 °C.

6. Procédé selon la revendication 5 caractérisé en ce que la température du milieu réactionnel est maintenue autour de 0 °C quand on commence à ajouter le formol et maintenue autour de 20 °C à la fin de l'introduction du formol.

7. Procédé selon la revendication 6 caractérisé en ce que la température du milieu réactionnel est portée, après introduction du formol, jusqu'à une température comprise entre 40 et 60 °C.

8. Procédé selon l'une quelconque des revendications 1 à 4 caractérisé en ce que la réaction est effectuée dans un solvant.

9. Procédé selon la revendication 8 caractérisé en ce que ledit solvant est choisi dans le groupe constitué par le toluène, le chlorure de méthylène, le chloroforme, le tétrachlorure de carbone.

10. Procédé selon l'une quelconque des revendications 8 ou 9 caractérisé en ce que la réaction est effectuée à une température comprise entre 30 et 60 °C.

## Claims

1. Process for the synthesis of chloromethyl chloroformate by the phosgenation of formol characterised in that gaseous, previously dried formol in the monomeric state is introduced into a reactor containing phosgene and a catalyst selected from the group consisting of substituted amides, tetra-substituted ureas or thioureas, phosphoramides completely substituted on the nitrogen atom(s), quaternary ammonium halides whose substituents include a total of at least 16 carbon atoms, alkali metal or alkaline earth metal halides associated with a complex of their cations, as well as the reaction products of these catalysts with phosgene, and in that the reaction is carried out in the complete absence of water and hydrochloric acid at a temperature of between − 10 °C and + 60 °C.

2. Process according to claim 1, characterised in that said catalyst is selected from the group consisting of substituted amides, tetra-substituted ureas or thioureas, phosphoramides completely substituted on the nitrogen atom(s), quaternary ammonium halides whose substituents each contain at least 4 carbon atoms, alkali metal or alkaline earth metal chlorides associated with a complex of their cations, as well as the reaction products of these catalysts with phosgene.

3. Process according to claim 2, characterised in that said catalyst is selected from the group consisting of dimethylformamide, tetrabutylurea, tetramethylurea, hexamethylphosphotriamide, tributylbenzyl ammonium chloride, potassium chloride associated with an 18-crown-6-ether and potassium chloride associated with (2.2.2.)-cryptate.

4. Process according to any one of claims 1 to 3 characterised in that the desiccating agent used for drying the formol is phosphorus pentoxide.

5. Process according to any one of claims 1 to 4, characterised in that during the introduction of formol the temperature of the reaction medium is maintained between − 10 °C and + 30 °C.

6. Process according to claim 5 characterised in that the temperature of the reaction medium is maintained at about 0 °C when the addition of formol is commenced and is maintained at a temperature of about 20 °C at the end of the formol introduction.

7. Process according to claim 6 characterised in that the temperature of the reaction medium is brought, after the introduction of formol, to a temperature of between 40 and 60 °C.

8. Process according to any one of claims 1 to 4 characterised in that the reaction is carried out in a solvent.

9. Process according to claim 8 characterised in that the solvent is selected from the group consisting of toluene, methylene chloride, chloroform and carbon tetrachloride.

10. Process according to any one of claims 8 to 9 characterized in that the reaction is carried out at a temperature of between 30 and 60 °C.

**Ansprüche**

1. Verfahren zur Herstellung von Chlorameisensäure-chlormethyl-estern durch Phosphenierung von Formaldehyd, dadurch gekennzeichnet, daß man vorher getrockneten, gasförmigen, monomeren Formaldehyd in einen Phosgen und einen Katalysator enthaltenden Reaktor einführt, wobei man den Katalysator aus der aus substituierten Amiden, tetrasubstituierten Harnstoffen oder Thioharnstoffen, am Stickstoff voll substituierten Phosphoramiden, quartären Ammoniumhalogeniden, deren Substituenten mindestens insgesamt 16 Kohlenstoffatome haben, Alkali- oder Erdalkalihalogeniden, die mit einem Komplexbildner für ihre Kationen assoziiert sind, sowie den Reaktionsprodukten dieser Katalysatoren mit dem Phosgen bestehenden Gruppe auswählt, die Reaktion in vollständiger Abwesenheit von Wasser und Chlorwasserstoff bei einer Temperatur von − 10 °C bis 60 °C durchführt.

2. Verfahren nach Anspruch 1, dadurch gekennzeichnet, daß man den Katalysator aus der aus substituierten Amiden, tetrasubstituierten Harnstoffen oder Thioharnstoffen, am Stickstoff voll substituierten Phosphoramiden, quartären Ammoniumhalogeniden, in denen jeder Substituent mindestens 4 Kohlenstoffatome hat, Alkali- oder Erdalkalichloriden, die mit einem Komplexbildner für ihre Kationen assoziiert sind, sowie den Reaktionsprodukten dieser Katalysatoren mit dem Phosgen bestehenden Gruppe auswählt.

3. Verfahren nach Anspruch 2, dadurch gekennzeichnet, daß man den Katalysator aus der aus Dimethylformamid, Tetrabutylharnstoff, Tetramethylharnstoff, Hexamethylphosphotriamid, Tributylbenzylammoniumchlorid, Kaliumchlorid, das an den 18-Kronenether-6 assoziiert ist, und Kaliumchlorid, das an Cryptat (2.2.2.) assoziiert ist, bestehenden Gruppe auswählt.

4. Verfahren nach einem der vorstehenden Ansprüche 1 bis 3, dadurch gekennzeichnet, dass das zum Trocknen des Formaldehyds verwendete Trockenmittel Phosphorpentoxid ist.

5. Verfahren nach einem der Ansprüche 1 bis 4, dadurch gekennzeichnet, dass die Temperatur des Reaktionsmediums während der Einführung des Formaldehyds von − 10 °C bis + 30 °C gehalten wird.

6. Verfahren nach Anspruch 5, dadurch gekennzeichnet, dass die Temperatur des Reaktionsmediums um etwa 0 °C am Beginn der Zugabe des Formaldehyds gehalten wird und um etwa 20 °C am Ende der Zugabe des Formaldehyds gehalten wird.

7. Verfahren nach Anspruch 6, dadurch gekennzeichnet, dass die Temperatur des Reaktionsmediums nach Zugabe des Formaldehyds bis auf eine Temperatur von 40 °C bis 60 °C gebracht wird.

8. Verfahren nach einem der Ansprüche 1 bis 4, dadurch gekennzeichnet, dass die Reaktion in einem Lösungsmittel durchgeführt wird.

9. Verfahren nach Anspruch 8, dadurch gekennzeichnet, dass das Lösungsmittel aus der aus Toluol, Methylenchlorid, Chloroform, Tetrachlorkohlenstoff bestehenden Gruppe ausgewählt wird.

10. Verfahren nach einem der vorstehenden Ansprüche 8 oder 9, dadurch gekennzeichnet, dass die Reaktion bei einer Temperatur von 30 bis 60 °C durchgeführt wird.